# EUROPEAN PATENT APPLICATION

(11) **EP 1 772 824 A2**
(43) Date of publication of application: **11.04.2007**
(21) Application number: 06020908.7
(22) Date of filing: 05.10.2006
(51) Int. Cl.: G06T 5/50

(54) **Method of processing an ultrasound image**

(30) Priority: 07.10.2005 KR 20050094313
(71) Applicant: MEDISON CO., LTD., Kangwon-do 250-870 (KR)
(72) Inventor: Kim, Cheol An, Discusser & Medison Bldg., 135-280 Seoul (KR); Ahn, Chi Young, Discusser & Medison Bldg., 135-280 Seoul (KR)
(74) Representative: Schmid, Wolfgang

(57) **Abstract**

The present invention is directed to a method of processing an ultrasound image, comprising the steps of: a) successively acquiring ultrasound images from an object; b) sampling ultrasound images in a predetermined time interval; c) determining an image difference between a currently sampled ultrasound image and a previously sampled ultrasound image; d) if the image difference is greater than a critical value, adjusting image parameters to optimize the image parameters; and e) applying the adjusted image parameters to the ultrasound images.

## Description

### FIELD OF THE INVENTION

The present invention generally relates to a method of processing an ultrasound image, and more particularly to a method of automatically adjusting image parameters to optimize the same and applying the adjusted image parameters to the ultrasound image.

### BACKGROUND OF THE INVENTION

An ultrasound diagnostic system has become an important and popular diagnostic tool since it has a wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., human organs).

The ultrasound diagnostic system generally uses a wide bandwidth transducer to transmit and receive ultrasound signals. The ultrasound diagnostic system forms images of human internal tissues by electrically exciting an acoustic transducer element or an array of acoustic transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which are amplified and processed to produce ultrasound data for an image of the tissues.

Further, since each of the human organs is positioned at different depth in the human body and has their inherent acoustic characteristics, an ultrasound image of each organ is displayed with different contrast and brightness. Therefore, it is required to optimally adjust image parameters such as gain, time gain control (TGC), dynamic range (DR) control, etc. according to the organ types or a scanning angle. This parameter control may be implemented by an image optimizing algorithm. However, since the image parameters must be adjusted by a user whenever the ultrasound image or the scanning angle is changed, the adjustment of image parameters can be highly inconvenient and very time consuming.

### SUMMARY OF THE INVENTION

The present invention is directed to providing a method of processing an ultrasound image by periodically checking a change in the ultrasound image and automatically adjusting image parameters to optimize the same.

In accordance with an aspect of the present invention, there is provided a method of processing an ultrasound image, comprising: a) successively acquiring ultrasound images from an object; b) sampling ultrasound images in a predetermined time interval; c) determining an image difference between a currently sampled ultrasound image and a previously sampled ultrasound image; d) if the image difference is greater than a critical value, adjusting image parameters to optimize the image parameters; and e) applying the adjusted image parameters to the ultrasound images.

### BRIEF DESCRIPTION OF THE DRAWINGS

The above and other objects and features of the present invention will become apparent from the following description of the preferred embodiments given in conjunction with the accompanying drawings, in which:
Fig. 1 is a flow chart showing a procedure for processing an ultrasound image in accordance with a preferred embodiment of the present invention;
Fig. 2 is a schematic diagram showing an example for explaining a method of comparing ultrasound frame images in accordance with the present invention; and
Fig. 3 is a schematic diagram showing an example for explaining a method of comparing ultrasound volume images in accordance with the present invention.

### DETAILED DESCRIPTION OF THE PRESENT INVENTION

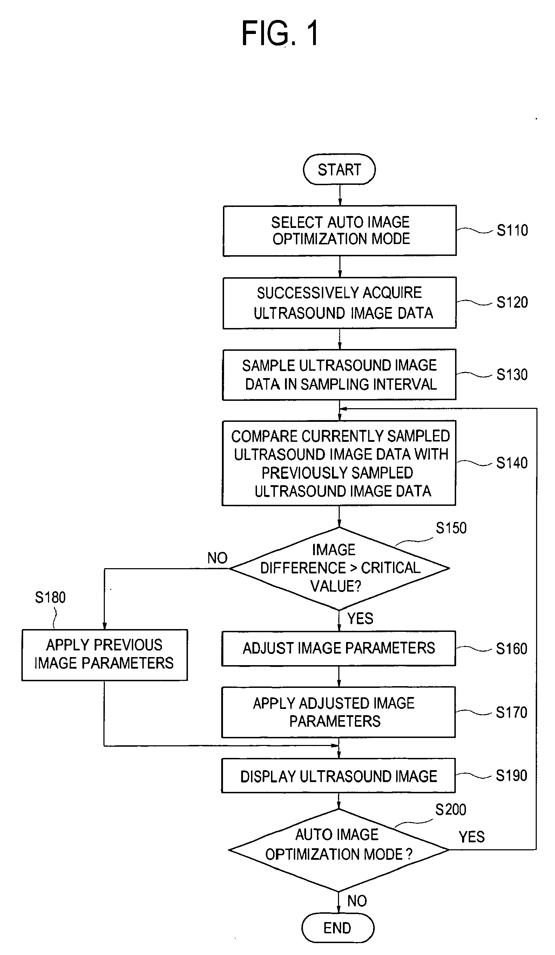

Fig. 1 is a flow chart showing a procedure for displaying an ultrasound image in accordance with a preferred embodiment of the present invention.

Referring to Fig. 1, if a sampling interval is set and then an auto image optimization mode is selected at step S110, then ultrasound image data are successively acquired from an object at step S120. The sampling interval represents a time interval to periodically sample an ultrasound image data from the successively acquired ultrasound image data. Further, the auto image optimization mode is a mode for automatically adjusting and optimizing image parameters such as gain, time gain, dynamic range (DR) to obtain an optimized ultrasound image. The sampling interval may be set within a range of 50 ms to 500 ms. The gain represents a value for adjusting amplification of a receive signal acquired from an ultrasound echo signal from the object. In addition, TG is a value for compensating a difference in strength of an ultrasound echo signal according to depth, whereas DR is a range of signal levels of receive signals acquired on the basis of the ultrasound echo signals reflected from the target object to be displayed. The ultrasound image data may be an ultrasound frame image data or an ultrasound volume image data.

Subsequently, the ultrasound image data are periodically sampled in the sampling interval at step S 130 and the sampled ultrasound image data are temporarily stored. Thereafter, a currently sampled ultrasound image data and a previously sampled ultrasound image data, which are stored, are compared to determine an image difference therebetween at step S 140. In order to compare the ultrasound images, each of the ultrasound images is segmented into a plurality of image blocks in a predetermined size and at least one image block is selected from each ultrasound image. In this case, the image blocks, which exist in an identical position at each ultrasound image, are selected. The selected block images are compared and an image difference resulting in the comparison result is checked to determine if it is greater than a critical value at step S150. The image difference may be calculated by comparing 1^{st} momentums such as averages and intermediates of pixels or voxels included in the selected block images or 2^{nd} momentums such as standard deviations of pixels or voxels included in the selected block images. Also, the image difference may be calculated by comparing the edge displacement of images between the selected block images. If the image difference is greater than the critical value at step S 150, then it is determined that the currently sampled ultrasound image is different from the previously sampled ultrasound image. That is, the ultrasound image is changed such that the image parameters are automatically adjusted to obtain optimized image parameters at step S160. The adjusted image parameters are applied to the current ultrasound image data at step S 170. On the other hand, if the image difference is equal to or less than the critical value, then it is determined that the compared ultrasound images are substantially identical to each other so that previously set image parameters are applied to the currently acquired ultrasound image at step S180. The ultrasound image, to which the image parameters are applied, is displayed at step S 190. Thereafter, it is checked whether a current process is in the auto image optimization mode at step S200. If it is determined the current process is in the auto image optimization mode, the steps S 140 to S 190 are repeated. On the other hand, if it is determined that the auto image optimization mode is cancelled, the process is ended.

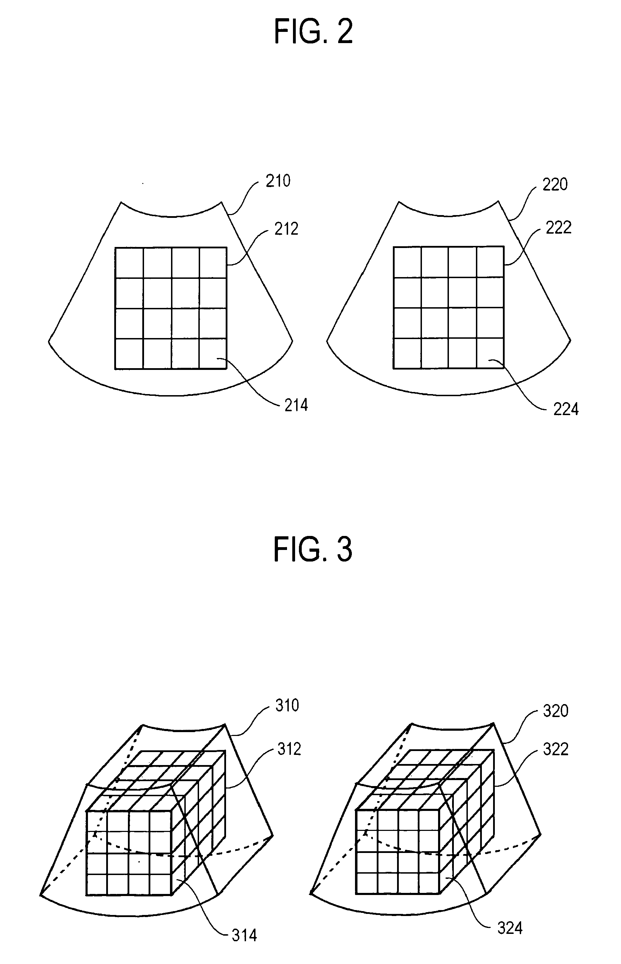

Fig. 2 is a schematic diagram showing an example for explaining a method of comparing ultrasound frame images in accordance with the present invention. As shown in Fig. 2, a currently sampled ultrasound frame image 210 and a previously sampled ultrasound frame image 220 are provided. A first box 212 and a second box 222 are set at a predetermined region on each of the ultrasound frame images 210 and 220, and each of the boxes 212 and 222 is segmented into a plurality of block images 214 and 224. The size of the first box 212 is set to be identical to that of the second box 222. Thereafter, at least one block is selected from each of the first and second boxes 212 and 222, respectively. In such a case, the blocks are identically positioned on the respective first and second boxes 212 and 222. Images existing in the selected blocks (hereinafter referred to as block images) are compared to determine the image difference therebetween by using 1^{st} momentums such as averages and intermediates of pixels included in the selected block images or 2^{nd} momentums such as standard deviations of pixels included in the selected block images. Also, the image difference may be calculated by comparing the edge displacement of images between the selected block images. If the image difference between the selected block images is greater than the critical value, then it is considered that the sampled ultrasound frame images are different from each other. In this case, the image parameters are automatically adjusted to obtain the optimized image parameters. On the other hand, if an image difference between the selected block images is equal to or less than the critical value, then it is considered that the sampled ultrasound frame images are substantially identical to each other. In such a case, previously set image parameters are applied to the ultrasound image to display an optimized ultrasound image.

Fig. 3 is a schematic diagram showing an example for explaining a method of comparing ultrasound volume images in accordance with the present invention. As shown in Fig. 3, a currently sampled ultrasound volume image 310 and a previously sampled ultrasound volume image 320 are provided. A first volume box 312 and a second volume box 322 are set at a predetermined region on each of the ultrasound volume images 310 and 320, respectively. Each of the volume boxes 312 and 322 is segmented into a plurality of volume block images 314 and 324. The size of the first volume box 212 is set to be identical to that of the second volume box 222. Thereafter, at least one volume block is selected from each of the first and second volume boxes 312 and 322. The volume images existing in the selected volume blocks (hereinafter referred to as volume block images) are compared by using 1^{st} momentums such as averages and intermediates of voxels included in the selected volume block images or 2^{nd} momentums such as standard deviations of voxels included in the selected volume block images. Also, the image difference may be calculated by comparing the edge displacement of images between the selected volume block images. If an image difference between the selected volume block images is greater than the critical value, then it is considered that the sampled ultrasound volume images are different from each other. In such a case, the image parameters are automatically adjusted to obtain optimized image parameters. On the other hand, if an image difference between the selected block images is equal to or less than the critical value, then it is considered that the sampled ultrasound frame images are substantially identical to each other. In this case, previously set image parameters are applied to the ultrasound image to display an optimized ultrasound image.

As mentioned above, the present invention allows the image parameters to be applied to the ultrasound image so as to be automatically adjusted, thus making it convenient to display an optimized ultrasound image.

While the present invention has been described and illustrated with respect to a preferred embodiment of the invention, it will be apparent to those skilled in the art that variations and modifications are possible without deviating from the broad principles and teachings of the present invention which should be limited solely by the scope of the claims appended hereto.

## Claims

1. A method of processing an ultrasound image, comprising the steps of:
a) acquiring ultrasound images successively from an object;
b) sampling the ultrasound images in a predetermined time interval;
c) determining an image difference between a currently sampled ultrasound image and a previously sampled ultrasound image;
d) if the image difference is greater than a critical value, adjusting image parameters to optimize the image parameters; and
e) applying the adjusted image parameters to the ultrasound images.

2. The method of Claim 1, further comprising the step of applying previously set image parameters to the ultrasound images if the image difference is equal to or less than the critical value.

3. The method of Claim 2, wherein the image parameters include gain, time gain and dynamic range.

4. The method of Claim 2, wherein the step c) comprises:
c1) segmenting the currently sampled ultrasound image and the previously sampled ultrasound image into a plurality of block images;
c2) selecting at least one block image from each of the segmented ultrasound images; and
c3) determining the image difference by comparing predetermined characteristics of images included in each of the selected block images.

5. The method of Claim 4, wherein the predetermined characteristics include averages and intermediates of pixels or voxels included in the selected block images or standard deviations of pixels or voxels included in the selected block images.

6. The method of Claim 4, wherein the predetermined characteristics include edge displacements of images between the selected block images.

7. The method of Claim 1, further comprising the step of storing the sampled ultrasound images.
